(19) Europäisches Patentamt
European Patent Office
Office européen des brevets

(11) EP 3 307 091 B1

# (12) EUROPEAN PATENT SPECIFICATION

(45) Date of publication and mention of the grant of the patent:
**14.10.2020 Bulletin 2020/42**

(21) Application number: **16726095.9**

(22) Date of filing: **31.05.2016**

(51) Int Cl.:
*A23L 33/165* (2016.01) *A23L 33/15* (2016.01)
*A23K 20/20* (2016.01) *A61K 31/295* (2006.01)
*A61P 3/02* (2006.01)

(86) International application number:
**PCT/EP2016/062267**

(87) International publication number:
**WO 2016/198285 (15.12.2016 Gazette 2016/50)**

(54) **FOOD COMPOSTION COMPRISING IRON NICOTINATE COMPLEX AND PHENOLIC CHROMOPHORE COMPOUNDS**

LEBENSMITTEL ENTHANLTEND EINEN EISEN-NICOTINAT-KOMPLEX UND PHENOLISCHE CHROMOPHORE VERBINDUNGEN

COMPOSITION ALIMENTAIRE COMPRENANT UN COMPLEXE DE NICOTINATE DE FER ET DES COMPOSÉS PHÉNOLIQUES CHROMOPHORES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.06.2015 EP 15171108**

(43) Date of publication of application:
**18.04.2018 Bulletin 2018/16**

(73) Proprietor: **Société des Produits Nestlé S.A.**
**1800 Vevey (CH)**

(72) Inventors:
- **HABEYCH NARVAEZ, Edwin Alberto**
  **1010 Lausanne (CH)**
- **GALAFFU, Nicola**
  **01210 Ornex (FR)**

(74) Representative: **Couzens, Patrick John**
**Société des Produits Nestlé S.A.**
**Avenue Nestlé 55**
**1800 Vevey (CH)**

(56) References cited:
**WO-A2-2006/037449 GB-A- 1 085 031**
**US-A- 4 216 144**

- **DATABASE BIOSIS [Online] BIOSCIENCES INFORMATION SERVICE, PHILADELPHIA, PA, US; May 2000 (2000-05), SHAIKHOVA G I ET AL: "Nutritious and biological value of "Temirli-non" flat-cake", XP002749030, Database accession no. PREV200100055784 & SHAIKHOVA G I ET AL: "Nutritious and biological value of "Temirli-non" flat-cake", UZBEKISTON TIBBIET ZHURNALI, no. 3, May 2000 (2000-05), pages 73-74,**
- **M Chylik: "Komplexna latka v terapii zelezom", , 1 April 1950 (1950-04-01), pages 282-288, XP55286025, Retrieved from the Internet: URL:http://www.chempap.org/file_access.php ? file=45-6a282.pdf [retrieved on 2016-07-05] & DATABASE CAPLUS, [Online] 1 January 1950 (1950-01-01), CHYLIK M ET AL: "The complex matter in therapeutics of iron", XP002749032, retrieved from CAPLUS; STN Database accession no. 1951-37119**
- **DATABASE CAPLUS [Online] CHEMICAL ABSTRACTS SERVICE, COLUMBUS, OHIO, US; 22 April 2001 (2001-04-22), Sidor: "Anemia in young pigs and its control by the compounds of iron and copper", XP002749031, retrieved from stn Database accession no. 1957:57810**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 307 091 B1

- DATABASE CAPLUS [Online] CHEMICAL ABSTRACTS SERVICE, COLUMBUS, OHIO, US; 14 June 2010 (2010-06-14), Gao: "Manufacture of slimming and face-nourishing beverage", XP002749033, retrieved from STN Database accession no. 2010:728204
- unknown: "A Water-insoluble Derivative of Nicotinic Acid for Food Enrichment, Ferrous Nicotinate", , 1944, XP002749034, Retrieved from the Internet: URL:https://books.google.nl/books/about/A_Water_insoluble_Derivative_of_Nicotini.html?id=98LXNwAACAAJ&redir_esc=y [retrieved on 2015-10-28]

## Description

### Field of the Invention

The present invention relates to a food composition comprising an iron nicotinate complex as well to the use of an iron nicotinate complex to fortify a food product with iron without colour change wherein the food product comprises phenolic chromophore compounds.

Billions of people around the world suffer from 'hidden hunger' or micronutrient malnutrition. They do not get enough of the micronutrients required to lead healthy productive lives from the foods that they eat. Micronutrients are vitamins and minerals (such as vitamin A, zinc, and iron) and are absolutely essential to good health. Micronutrient malnutrition can lower IQ, cause stunting and blindness in children, lower resistance to disease in both children and adults, and increase risks for both mothers and infants during childbirth. Iron deficiency is the most common and widespread nutritional disorder in the world. As well as affecting a large number of children and women in developing countries, it is the only nutrient deficiency which is also significantly prevalent in industrialized countries. In wealthier countries people may voluntarily choose a diet which may cause a reduced iron intake, such as a vegetarian diet. Food fortification is one method to increase iron intake along with dietary diversification and enhancement of iron absorption.

The main problems caused by iron sources added to foods are colour and off-flavour production, especially in the presence of oxygen, light and at high temperature. If highly or slightly soluble sources of iron are used, interaction between the iron and iron sensitive ingredients, such as polyphenols, occurs. Thus, the addition of ferrous sulphate or other soluble iron salts such as ferric sulphate, ferrous lactate, ferrous gluconate, ferrous fumarate, ferric citrate, ferric ammonium citrate, etc., to foods compositions such as chocolate powders and other RTD (ready-to-drink) mixes causes them to turn dark grey when reconstituted with water or milk. Food compositions containing fruits and vegetables are also sensitive to colour change.

Unfortunately, fortifying foods with iron can lead to a number of undesirable changes in the properties of the food, for example, and it can lead to unwanted colour changes in food.

Another problem in iron fortification is the capacity of iron to promote destructive free-radical reactions, which can result in off-flavours. Thus, the addition of soluble iron sources to fat containing products - mostly products with a high level of unsaturated fatty acids - cause flavour changes due to lipid oxidation. Iron promoted oxidation not only affects the organoleptic properties of foods, but also undesirably affects the nutritional quality of these products. Iron can accelerate oxidation reactions destroying nutrients such as essential fatty acids and vitamins. Undesirable interactions with iron can be also enhanced during heat treatment, such as pasteurization or sterilization.

Iron may be provided in less reactive forms such as elemental iron or ferric pyrophosphate. This reduces the problem of undesirable reactions in food compositions, but these less reactive forms of iron are also less bioavailable. It would be desirable to provide iron sources which have good bioavailability whilst also not causing undesirable changes when added to food compositions.

EP1011344 describes chocolate-flavoured beverage mixes and other edible mixes that are fortified with sources of iron such as ferrous fumarate and ferrous sulphate, yet do not develop undesirable grey colour when the beverage mix is reconstituted with aqueous liquids including fruit juice. The problem of grey colour development is solved by including edible acids such as citric or malic acid as buffering agents in the beverage mix so that the pH of the reconstituted chocolate beverage is about 6.5 or less. Controlling the pH to be acidic does not always suit the desired taste of the product. Also, controlling the pH is generally most suitable for beverages, where any coloured components are generally dissolved or dispersed in a continuous aqueous phase and so can be influenced by added acids. For non-beverage food compositions it may be difficult to ensure that all the components responsible for the development of an undesirable colour are affected by added acids.

DATABASE CAPLUS [online] CHEMICAL ABSTRACTS SERVICE, (2010-06-14), Database accession no. 2010:728204, GAO: "Manufacture of slimming and face-nourishing beverage", discloses a beverage containing various plant extracts and iron nicotinate.

U.S. patent 4,216,144 describes an iron proteinate coordination complex with at least three protein hydrolysate ligands selected from the group consisting of polypeptides, peptides and naturally occurring amino acids. The iron proteinate has been shown by analysis to be at least 70 to 80 percent chelated. Therefore, because of 20 to 30% of free iron, the patented chelate cannot prevent discoloration and off-flavor development, particularly when it is used to fortify polyphenol or other fat containing products, such as cocoa, coffee, tea, banana, etc. Thus, improvements in this area are needed.

There remains a need to provide improved or alternative food compositions which provide a nutritional source of iron without exhibiting undesirable changes such as colour or flavour changes. In particular there is a need to provide food compositions which provide iron with good bioavailability, stabilized by ingredients with wide consumer acceptability and which provide nutritional value themselves.

Any reference to prior art documents in this specification is not to be considered an admission that such prior

art is widely known or forms part of the common general knowledge in the field. As used in this specification, the words "comprises", "comprising", and similar words, are not to be interpreted in an exclusive or exhaustive sense. In other words, they are intended to mean "including, but not limited to".

## Summary of the Invention

An object of the present invention is to improve the state of the art and to provide a solution to overcome at least some of the inconveniences described above or at least to provide a useful alternative. The object of the present invention is achieved by the subject matter of the independent claims. The dependent claims further develop the idea of the present invention.

Accordingly, an aspect of the present invention provides a food composition comprising an iron nicotinate complex.

The inventors have found that iron added to food compositions in the form of ferrous nicotinate causes less colour change than iron added as ferrous sulphate. This is particularly surprising as ferrous nicotinate, like ferrous sulphate, is fully soluble in water and therefore will have good bioavailability. Iron nicotinate complexes such as ferrous nicotinate therefore provide good stability in iron-containing food compositions and have good bioavailability.

## Detailed Description of the invention

Consequently the present invention relates in part to a food composition comprising an iron nicotinate complex. The food composition may be fortified in iron. A food composition fortified in iron is a food composition in which the content of iron has been increased, normally to improve its nutritional quality. The iron may, or may not have been originally in the food. Iron may be added in the form of Fe(II) or Fe(III) ions.

Nicotinic acid (pyridine-3-carboxylic acid) is also known as niacin or vitamin $B_3$. Having enough nicotinic acid in the body is important for general good health. Higher amounts of nicotinic acid can improve cholesterol levels and lower cardiovascular risks. It is particularly advantageous that, when combined in a complex with iron, nicotinate provides stable food compositions with good iron bioavailability but also is an important nutrient in its own right.

The iron nicotinate complex may be ferrous nicotinate, for example it may have the structure shown in (I) below.

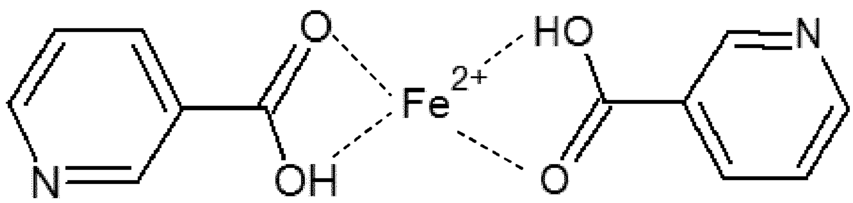

(I)

Ferrous nicotinate is commercially available, for example it may be obtained from MP Biomedicals of Illkirch in France. The iron nicotinate complex may be exogenously synthesized. The iron nicotinate may be added to the food as ferrous nicotinate tetrahydrate. The iron nicotinate complex may be present at a level providing at least 2 ppm of iron in the food composition.

Unwanted colour changes in fortified food compositions may be caused by iron forming complexes with chromophore compounds, for example phenolic chromophore compounds. A chromophore is a region of a molecule where the energy difference between two different molecular orbitals is such that visible light can be absorbed by exciting an electron from its ground state into an excited state. Phenolic chromophore compounds have at least one phenol structure as part of the chromophore. Examples of phenolic chromophore compounds include anthocyanins, anthocyanidins, betanin, gallic acid, curcumin and carminic acid. Non-phenolic chromophore compounds include chlorophyll.

The interaction between iron ions and chromophores can cause a change in the maximum absorption wavelength. Typically this is a change to a longer wavelength, such a change being referred to as a bathochromic shift. The light absorption intensity may also increase; this is the hyperchromic effect. For food comprising chromophore compounds, a bathochromic shift due to the addition of iron causes the food to change colour, which is generally undesirable.

The problem of colour change when fortifying food with iron is particularly apparent with food compositions containing fruit or vegetables as these are generally rich in chromophore compounds such as phenolic chromophore compounds. The addition of iron leads to a bathochromic shift in the colour of the chromophore compounds in the fruit

or vegetables resulting in an undesirable change in the colour of the food. In some cases, chromophores which did not have a significant influence on the colour of the fruit or vegetables before the addition of iron, produce an undesired colour when in the presence of iron.

Fruits and vegetables intrinsically provide a good source of beneficial dietary nutrients, and so are good ingredients for delivering additional nutritional benefits to food. The food composition of the current invention may further comprise fruit or vegetables. With the iron in the form of iron nicotinate complex, problems of colour change in food compositions comprising fruit or vegetables are avoided or reduced.

The fruit may, for example, be in the form of fresh fruit, fresh fruit pieces, fruit powder, dried fruit, or fruit purée. Fruit provides beneficial dietary nutrients, such as vitamins and minerals together with dietary antioxidants such as polyphenols. These strong nutritional credentials make food compositions comprising fruit a suitable vehicle for further fortification, such as with iron. Fruit can also add attractive texture and colour to food compositions. The fruit may be selected from the group consisting of strawberry, raspberry, blueberry, blackberry, apricot, pear, banana, quince, wolfberry and mixtures of these.

The fruit may be a culinary fruit. The culinary sense of the term fruit includes fleshy seed-associated structures of a plant that are sweet and edible in the raw state, such as apples, oranges, grapes, strawberries and bananas. Culinary fruits do not include some foods which are fruit in a botanical sense; for example, beans, nuts and cereal grains.

The food composition of the invention may comprise fruit or vegetables at a level of at least 1 wt.% in the food composition, for example at a level of at least 2 wt.%, for further example at a level of at least 5 wt.%. The maximum level of fruit may be close to 100 wt.%, for example a fruit puree fortified by iron nicotinate at 0.015% would contain 99.985 wt.% fruit if there were no other ingredients. For processed fruits or vegetables such as dried fruit or vegetable powder, 1 wt.% means 1% by weight of fresh fruit or vegetable equivalent. The fruit may be selected from the group consisting of strawberry, raspberry, blueberry, blackberry, apricot, pear, banana, quince, wolfberry and mixtures of these.

The food composition of the current invention may comprise phenolic chromophore compounds. The phenolic chromophore compounds of the invention may be selected from the group consisting of curcumin; carminic acid; polyphenols, including anthocyanins, anthocyanidins and gallic acid; and mixtures of these. These compounds are commonly found in foods. The phenolic chromophore compounds may be comprised within other food materials, for example gallic acid in banana puree, anthocyanins in blueberries or curcumin in turmeric. Alternatively, the phenolic chromophore compounds may be added directly as a colour, for example carminic acid added in the form of carmine which is an aluminium salt of carminic acid commonly used as a food colour. The greater the quantity of phenolic chromophore compounds in a food composition, the greater the potential colour change will be in the presence of iron. The phenolic chromophore compounds may be present in an amount between 0.0005 and 5 wt% of the composition.

Problems of colour change in iron-containing food compositions frequently occur over time, reducing the acceptable shelf-life of the product, or they occur when the product is heated, for example during a sterilization or pasteurization processes. The food composition of the invention may have been heat treated. The invention provides a means to prevent undesirable colour changes during heat treatment. The food composition of the invention may have a ΔEab* value less than 2.5, for example less than 2, after a heat treatment of 2 minutes at 105 °C.

The CIE 1976 L*a*b* (hereinafter CIELAB) colour scale is one method of measuring colour proposed by the Commission Internationale de l'Éclairage (CIE) [CIE Technical Report, Colorimetry 2nd Edition, CIE 15.2 - 1986, corrected reprint 1996]. The CIELAB colour space is produced by plotting the quantities L*, a*, b* in rectangular coordinates. The L* coordinate of an object is the lightness intensity as measured on a scale from 0 (black) to 100 (absolute white). The a* and b* coordinates have no specific numerical limits. The parameter a* runs from pure green (negative a*) to pure red (positive a*), while b* runs from pure blue (negative b*) to pure yellow (positive b*).

In the CIELAB colour space, colour difference may be calculated as a single value taking into account the differences between the L*, a* and b* values of two samples. The colour difference ΔEab* is calculated as follows:

$$\Delta Eab^* = \sqrt{(\Delta L^*)^2 + (\Delta a^*)^2 + (\Delta b^*)^2}$$

The food composition of the invention may be a beverage or non-beverage food product. The food composition of the invention may be a biscuit; a cereal bar; a breakfast cereal; a dairy product; a beverage, for example a powdered beverage or a ready-to-drink beverage; a culinary product (for example a concentrated stock or dehydrated stock); a nutritional supplement or a pet food. All of these products may pose problems of colour change when fortified by iron. For example biscuit, cake and pastry products may be coloured by natural colours such as anthocyanins or carmine; the products may have coloured fillings or coatings. Breakfast cereals may contain fruit, for example fruit inclusions or fruit fillings. Cereal bars may contain coloured fruit such as cranberries, or have coloured inclusions containing added vitamins and minerals, such as small chewy pieces of jelly. Ice creams and desserts may be coloured by anthocyanins, particularly when fruit flavoured. Prepared meals often contain vegetables and nutritional supplements may contain fruit or vegetables for example in the form of fruit or vegetable powder, or may be coloured by the addition of natural colours

to make them more appealing. Pet foods such as dog treats may contain fruit, for example berries. All these products may be sensitive to colour change on addition of iron. It is therefore an advantage that iron nicotinate may be used in these products to achieve iron fortification whilst preventing colour change, for example colour change caused by a bathochromic shift.

The food composition of the invention may be a concentrated stock or dehydrated stock, for example a bouillon cube. Concentrated stock is used around the world as a basic cooking ingredient. Stock, in the form of bouillon cubes, is a relatively cheap ingredient and so is popular in developing countries. This makes bouillon cubes a suitable carrier for iron, to address iron-deficiency in developing countries. However, many concentrated stocks, especially vegetable or chicken stocks, contain phenolic chromophore compounds, such as curcumin in turmeric or cyanidin in red onions. In the presence of iron, these phenolic chromophore compounds change colour due to a bathochromic shift. The colour change is generally undesirable. In a bouillon cube the colour change may manifest itself when the bouillon is rehydrated with hot water. For example, a curcumin-containing stock may change colour from yellow to blue. It is therefore one of the benefits of the invention that iron fortification of concentrated or dehydrated stock with iron nicotinate avoids this colour change.

The food composition of the invention may be yoghurt or may comprise yoghurt. The yoghurt may be a spoonable yoghurt or a drinking yoghurt. Yoghurt is a good source of calcium, helping to form and maintain strong bones. Yoghurt may also be fortified with other beneficial minerals such as magnesium and zinc. However, fortifying yoghurt with iron presents a problem if the yoghurt contains phenolic chromophore compounds, such as may be found in yoghurts containing fruit. For example, a blueberry yoghurt, coloured by the anthocyanins in blueberries, will change colour after addition of iron; the anthocyanins undergoing a bathochromic shift. Similarly a banana yoghurt, which was initially a pale yellow colour, may develop an unattractive grey-blue colour on addition of iron. Bananas comprise polyphenols such as gallic acid, catechin, epicatechin and epigallocatechin. It is an advantage that the invention provides iron fortified food compositions comprising yoghurt where these colour change problems do not occur. In the context of the present invention the term yoghurt may include, but is not limited to, materials complying with local food labelling regulations concerning the term "yoghurt". For example the food composition according to the invention may be an acidified dairy beverage.

The food composition of the invention may comprise polyunsaturated fatty acids. The polyunsaturated fatty acids in the food composition of the invention may be short chain polyunsaturated fatty acids (SC-PUFA), for example the essential fatty acids alpha-linolenic acid (an omega-3 fatty acid) or linoleic acid (an omega-6 fatty acid). The polyunsaturated fatty acids in the food composition of the invention may be long chain polyunsaturated fatty acids (LC-PUFA), for example eicosapentaenoic acid, docosahexaenoic acid, gamma-linolenic acid, dihomo-gamma-linolenic acid or arachidonic acid. Providing the iron of the food composition of the invention as an iron nicotinate complex prevents or reduces the oxidation of polyunsaturated fatty acids by iron. The polyunsaturated fatty acids may be present in an amount between 0.02 to 10 % by weight of the composition.

The food composition of the invention may other micronutrients, for example the food composition may comprise vitamin A and/or zinc.

In a further aspect, the invention provides a food composition comprising an iron nicotinate complex for use in the treatment or prevention of iron deficiency. Iron nicotinate complexes have excellent solubility in water and so provide a bioavailable form of iron. The food composition for use according to the invention may comprise an iron nicotinate complex present at a level providing at least 6 ppm of iron in the composition, for example at least 8 ppm of iron in the composition, for example at least 8 ppm of iron in the composition. An iron nicotinate complex present in the food composition at a level providing these amounts of iron provides an effective level of iron for treatment or prevention of iron deficiency. The recommended daily allowance for iron intake in humans varies from 8 to 18 mg per day depending on age and sex. Children, women up to the time of menopause, and expectant and nursing mothers are in the group with higher requirements of iron. The food composition for use according to the invention may provide at a daily dose of 0.1 to 0.5 mg iron per kg of body weight.

In a further aspect, the invention provides the use of an iron nicotinate complex to fortify a food product with iron. The use of an iron nicotinate complex according to the invention may be to fortify a food product without colour change. Some changes in appearance can be detected by appropriate instruments but would not be of concern to someone consuming a product. The term "colour change" in this specification may be considered to be a difference in colour of sufficient magnitude that a human observer would perceive the difference between two samples shown one after the other but not simultaneously. For example, a colour change may be considered to be a CIELAB $\Delta Eab^*$ colour difference greater than 2, for example greater than 3. A food product fortified with iron is a food product in which the content of iron has been increased in the food, normally to improve its nutritional quality. The iron may, or may not have been originally in the food. Iron may be added in the form of Fe(II) or Fe(III) ions.

An iron nicotinate complex may be used according to the invention to prevent colour change over the product's shelf-life. For example, the CIELAB $\Delta Eab^*$ colour difference between an fortified food product at the time of its manufacture and the end of its shelf-life under recommended storage conditions may be less than 3, for example less than 2. Shelf

life is the recommended length of time that foods, beverages, and many other perishable items can be stored during which the defined quality of a specified proportion of the goods remains acceptable under expected (or specified) conditions of distribution, storage and display. Typically a "best before date" (BBD) is printed on packaged perishable foods together with recommended storage conditions. Where such a BBD is indicated, the shelf-life is the time between manufacture and the BBD. Where a BBD is not indicated, the shelf-life is the equivalent period usual for the relevant product type.

An iron nicotinate complex may be used according to the invention to prevent colour change during heat treatment. For example, the iron fortified food product may have a ΔEab* value less than 3, for example less than 2.5, after a heat treatment of 2 minutes at 105 °C.

A further aspect of the invention is a process for fortifying a food product with iron comprising preparing a food product and adding an iron nicotinate complex. Such a process has the advantage of producing an iron fortified product which provides a bioavailable source iron without exhibiting undesirable changes such as colour or flavour changes. The process for fortifying the food product with iron may further comprise a heat treatment step, for example heat treatment to reduce or eliminate food spoilage organisms.

Those skilled in the art will understand that they can freely combine all features of the present invention disclosed herein. In particular, features described for the product of the present invention may be combined with the use of the present invention and vice versa. Further, features described for different embodiments of the present invention may be combined. Where known equivalents exist to specific features, such equivalents are incorporated as if specifically referred to in this specification. Further advantages and features of the present invention are apparent from the non-limiting examples.

## Examples

Example 1: Iron fortified banana fermented milk product

Nestle Jogolino™ Banana fermented milk product containing 47.3 % banana puree, was iron fortified by the addition of different iron containing materials so as to provide approximately 2.1 mg iron per 100g yoghurt. A 100g serving with that level of iron would provide 35 % of the recommended daily amount of iron for a young child [Official Journal of the European Union, Commission Directive 2006/125/EC].

The milk products were flash pasteurized at 105 °C for 2 minutes. Colour measurements were performed in 1x1 cm polystyrene cuvettes using an X-Rite ColorEye 7000A colorimeter. The colorimeter was set up with a D65 light source, 10 degree observer angle and with specular component included. The colour difference between the product with no iron salts and the iron fortified product was measured for each iron salt and expressed as ΔEab* using the CIELAB colour scale.

| Trial | Fe material | ΔEab* |
|---|---|---|
| A | Ferrous sulphate | 3.26 |
| B | Ferric pyrophosphate | 3.36 |
| C | Ferrous nicotinate | 2.11 |

The product fortified with ferrous nicotinate showed the smallest colour change on pasteurization.

## Claims

1. Food composition comprising an iron nicotinate complex comprising fruits comprising chromophore compounds wherein the fruits are selected from the group consisting of strawberry, raspberry, blueberry, blackberry, apricot, pear, banana, quince and mixtures of these.

2. A food composition according to claim 1 wherein the phenolic chromophore compounds are selected from the group consisting of polyphenols, including anthocyanins, anthocyanidins and gallic acid; and mixtures of these.

3. A food composition according to any one of claims 1 to 2 wherein the food composition has been heat treated.

4. A food composition according to any one of claims 1 to 3 wherein the ΔEab* value of the food composition is less

than 2.5 after a heat treatment of 2 minutes at 105 °C.

**5.** A food composition according to any one of claims 1 to 4 wherein the food composition is a biscuit; a cereal bar; a breakfast cereal; a dairy product; a beverage; a culinary product; a nutritional supplement or a pet food.

**6.** A food composition according to any one of claims 1 to 5 further comprising polyunsaturated fatty acids.

**7.** A food composition according to any one of claims 1 to 6 further comprising vitamin A and/or zinc.

**8.** Use of an iron nicotinate complex to fortify a food product with iron without colour change wherein the food product comprises phenolic chromophore compounds.

**9.** Use of an iron nicotinate complex according to claim 8 wherein colour change during heat treatment is prevented.

**Patentansprüche**

**1.** Nahrungsmittelzusammensetzung, umfassend einen Eisen-Nicotinat-Komplex, umfassend Früchte, die chromophore Verbindungen umfassen, wobei die Früchte ausgewählt sind aus der Gruppe bestehend aus Erdbeere, Himbeere, Blaubeere, Brombeere, Aprikose, Birne, Banane, Quitte und Mischungen davon.

**2.** Nahrungsmittelzusammensetzung nach Anspruch 1, wobei die phenolischen chromophoren Verbindungen ausgewählt sind aus der Gruppe bestehend aus Polyphenolen, einschließlich Anthocyaninen, Anthozyanidinen und Gallussäure; und Mischungen davon.

**3.** Nahrungsmittelzusammensetzung nach einem der Ansprüche 1 bis 2, wobei die Nahrungsmittelzusammensetzung wärmebehandelt wurde.

**4.** Nahrungsmittelzusammensetzung nach einem der Ansprüche 1 bis 3, wobei der ΔEab*-Wert der Nahrungsmittelzusammensetzung nach einer 2-minütigen Wärmebehandlung bei 105 °C weniger als 2,5 beträgt.

**5.** Nahrungsmittelzusammensetzung nach einem der Ansprüche 1 bis 4, wobei die Nahrungsmittelzusammensetzung ist: ein Keks; ein Getreideriegel; eine Frühstückszerealie; ein Milchprodukt; ein Getränk; ein kulinarisches Produkt; ein Nahrungsergänzungsmittel oder eine Tiernahrung.

**6.** Nahrungsmittelzusammensetzung nach einem der Ansprüche 1 bis 5, ferner umfassend mehrfach ungesättigte Fettsäuren.

**7.** Nahrungsmittelzusammensetzung nach einem der Ansprüche 1 bis 6, ferner umfassend Vitamin A und/oder Zink.

**8.** Verwendung eines Eisen-Nicotinat-Komplexes zum Anreichern eines Nahrungsmittelprodukts mit Eisen ohne Farbänderung, wobei das Nahrungsmittelprodukt phenolische chromophore Verbindungen umfasst.

**9.** Verwendung eines Eisen-Nicotinat-Komplexes nach Anspruch 8, wobei die Farbänderung während der Wärmebehandlung verhindert wird.

**Revendications**

**1.** Composition alimentaire comprenant un complexe de nicotinate de fer comprenant des fruits comprenant des composés chromophores dans laquelle les fruits sont choisis dans le groupe constitué de fraise, framboise, myrtille, mûre, abricot, poire, banane, coing et mélanges de ceux-ci.

**2.** Composition alimentaire selon la revendication 1 dans laquelle les composés chromophores phénoliques sont choisis dans le groupe constitué de polyphénols, y compris anthocyanines, anthocyanidines et acide gallique ; et des mélanges de ceux-ci.

**3.** Composition alimentaire selon l'une quelconque des revendications 1 à 2 dans laquelle la composition alimentaire

a été traitée thermiquement.

4. Composition alimentaire selon l'une quelconque des revendications 1 à 3 dans laquelle la valeur ∆Eab* de la composition alimentaire est inférieure à 2,5 après un traitement thermique de 2 minutes à 105 °C.

5. Composition alimentaire selon l'une quelconque des revendications 1 à 4 dans laquelle la composition alimentaire est un biscuit ; une barre de céréales ; une céréale de petit-déjeuner ; un produit laitier ; une boisson ; un produit culinaire ; un complément nutritionnel ou un aliment pour animal de compagnie.

6. Composition alimentaire selon l'une quelconque des revendications 1 à 5 comprenant en outre des acides gras polyinsaturés.

7. Composition alimentaire selon l'une quelconque des revendications 1 à 6 comprenant en outre de la vitamine A et/ou du zinc.

8. Utilisation d'un complexe de nicotinate de fer pour enrichir en fer un produit alimentaire sans changement de couleur dans laquelle le produit alimentaire comprend des composés chromophores phénoliques.

9. Utilisation d'un complexe de nicotinate de fer selon la revendication 8 dans laquelle un changement de couleur pendant un traitement thermique est empêché.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1011344 A
- US 4216144 A


### Non-patent literature cited in the description


- CIE Technical Report, Colorimetry. CIE, 1986, vol. 15.2
- *Official Journal of the European Union*